# EUROPÄISCHE PATENTSCHRIFT

(11) **EP 1 751 095 B1**
(45) Veröffentlichungstag und Bekanntmachung des Hinweises auf die Patenterteilung: **18.06.2014**
(21) Anmeldenummer: 05745589.1
(22) Anmeldetag: 06.05.2005
(51) Int. Cl.: C07D 209/18, C07C 237/24

(54) **SUBSTITUIERTE CYCLOHEXYLCARBONSÄURE-DERIVATE**
SUBSTITUTED CYCLOHEXYLCARBOXYLIC ACID DERIVATIVES
DERIVES D'ACIDE CYCLOHEXYLE-CARBOXYLIQUE SUBSTITUES

(30) Priorität: 10.05.2004 DE 102004023632
(43) Veröffentlichungstag der Anmeldung: 14.02.2007
(73) Patentinhaber: Grünenthal GmbH, 52078 Aachen (DE)
(72) Erfinder: HINZE, Claudia, 53639 Königswinter (DE); SUNDERMANN, Bernd, 61381 Friedrichsdorf (DE); SCHICK, Hans, 13086 Berlin-Weissensee (DE)
(74) Vertreter: Bülle, Jan
(86) Internationale Anmeldenummer: PCT/EP2005/004908
(87) Internationale Veröffentlichungsnummer: WO 2005/110977

(56) Entgegenhaltungen:
- EP-A- 1 325 910
- WO-A-01/87838

## Beschreibung

Die vorliegende Erfindung betrifft substituierte Cyclohexylcarbonsäure-Derivate, Verfahren zu deren Herstellung, Arzneimittel enthaltend diese Verbindungen und die Verwendung von substituierten Cyclohexylcarbonsäure-Derivate-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische µ-Opioide wie Morphin sind bei der Therapie starker bis stärkster Schmerzen gut wirksam und von größter Bedeutung für die Schmerztherapie. Es kann jedoch von Vorteil sein, wenn neben dem µ-Opioid-Rezeptor auch andere Opioid-Rezeptoren, insbesondere der ORL-1-Rezeptor, beeinflusst werden, da die reinen µ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application, Wiley VCH, 2002).

Darüber hinaus ist bekannt, dass eine sich eine Beeinflussung der Serotonin- und/oder Noradrenalin-Wiederaufnahme günstig auf das Wirk- und Nebenwirkungsspektrum von Opioiden auswirken kann (Beispiel: Tramadol, s. Opioids with Clinical Relevance; Tramadol, 228-230 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al. Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Simulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Aus dem Stand der Technik (WO 02090317) sind strukturell verwandte Verbindungen bekannt, die eine Affinität zum ORL-1-Rezeptor besitzen. Für diese Strukturklasse wurde bislang kein Einfluss auf die Noradrenalin- und Serotonin-Wiederaufnahme beschrieben.

Aus dem Stand der Technik (EP 1325910 A) sind weiterhin aliphatische, stickstoff-haltige, fünfgliedrige Ringverbindungen bekannt, die eine inhibierende Wirkung auf Dipeptidylpeptidase IV (DPPIV) haben. WO 98/19998 und WO 00/34241 offenbaren ähnliche Verbindungen mit inhibierender Wirkung auf DPPIV.

Weiterhin sind geminal disubstituierte Cyclohexanderivate bekannt, die als Tachykinin-Antagonisten wirken (WO 01/87838).

Aufgabe der vorliegenden Erfindung war es, Arzneimittel zur Verfügung zu stellen, die auf das Opioid-Rezeptor-System wirken und damit für Arzneimittel, insbesondere zur Behandlung der verschiedenen mit diesem System nach dem Stand der Technik in Verbindung stehenden Krankeiten bzw. zum Einsatz in den dort genannten Indikationen, geeignet sind. Daüber hinaus sollten die Verbindungen die Noradrenalin- und Serotonin-Wiederaufnahme beeinflussen.

Gegenstand der Erfindung sind daher substituierte Cyclohexylcarbonsäure-Derivate-Derivate der allgemeinen Formel I, 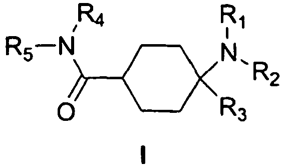 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; über C₁₋₃-Alkyl-Gruppe gebundenes Aryl, Heteroaryl oder C₃₋₈-Cycloalkyl, jeweils unsubstituiert oder einfach oder mehrfach substituiert; Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl, steht;
R⁴ für für-(CR⁶R⁷)ₙR⁸ steht,
wobei n 0, 1, 2, 3, 4, 5 oder 6 bedeutet
R⁶ H oder C₁₋₅-Alkyl, gesättigt oder ungesättigt, verzweigt oder unverzweigt, unsubstituiert oder einfach oder mehrfach substituiert, bedeutet
R⁷ H, C₁₋₅-Alkyl, gesättigt oder ungesättigt, verzweigt oder unverzweigt, unsubstituiert oder einfach oder mehrfach substituiert, oder COOR⁹ bedeutet,
oder R⁶ und R⁷ einen Ring (CH₂)ₖCHR⁸(CH₂)ₘ bilden, mit k = 1, 2, 3 und m = 1, 2
R⁸ Cycloalkyl, Aryl oder Heteroaryl, jeweils unsubstituiert oder einfach oder mehfach substituiert, bedeutet,
R⁹ H oder C₁₋₅-Alkyl, gesättigt oder ungesättigt, verzweigt oder unverzweigt, unsubstituiert oder einfach oder mehrfach substituiert, bedeutet
R⁵ für H oder für-(CH₂)ₗR⁸ steht,
wobei I für 1, 2 oder 3 steht,
oder zusammen mit R⁴ für CH₂CH₂OCH₂CH₂ oder CH₂CH₂NR¹¹CH₂CH₂ stehen,
wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Verbindungen zeigen gute Bindung an den µ-Rezeptor und den ORL-1-Rezeptor, aber auch an andere Opioidrezeptoren. Überraschenderweise zeigte es sich, dass die Verbindungen auch gute Inhibitoren der Noradrenalin- und der Serotonin-Wiederaufnahme sind. Damit eignen sie sich auch zur Behandlung von Depressionen, und/oder Bulimie und/oder Anorexie und/oder Katalepsie und/oder zur Anxiolyse und/oder zur Vigilanz- und/oder Libidosteigerung.

Die Ausdrücke "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl (-CH₂CH=CH₂, -CH=CH-CH₃, -C(=CH₂)-CH₃), Propinyl (-CH-C=CH,-C≡C-CH₃), 1,1-Dimethylethyl, 1,1-Dimethylpropyl, Butenyl, Butinyl, Pentenyl und Pentinyl, umfaßt.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (-CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff/Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, =O, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryₗ, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂, Si(C₁₋₆-Alkyl)₃, Si(C₃₋₈-Cycloalkyl)₃, Si(CH₂-C₃₋₈-Cycloalkyl)₃, Si(Phenyl)₃, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH,

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryₗ, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mendelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen substituierten Cyclohexylcarbonsäure-Derivate gilt, dass
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₅-Alkyl, gesättigt oder ungesättigt, verzweigt oder unverzweigt, einfach oder mehrfach substituiert oder unsubstituiert, bedeutet.

Besonders bevorzugt sind substituierte Cyclohexylcarbonsäure-Derivate, worin R¹ und R² unabhängig voneinander für CH₃ oder H stehen, wobei R¹ und R² nicht gleichzeitig H bedeuten.

Weiterhin bevorzugt sind substituierte Cyclohexylcarbonsäure-Derivate, worin
R³ für Cyclopentyl, Cyclohexyl, Naphthyl, Thiophenyl, Benzothiophenyl, Furyl, Benzofuranyl, Benzodioxolanyl, Indolyl, Indanyl, Benzodioxanyl oder Pyridyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4-Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl steht;
insbesondere
R³ für Naphthyl, Thiophenyl oder Pyridyl, jeweils unsubstituiert oder einfach oder mehrfach substituiert; über eine gesättigte, unverzweigte C₁₂-Alkyl-Gruppe gebundenen C₅₋₆-Cycloalkyl, Phenyl, Naphthyl, Thiophenyl, Pyridyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorophenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl oder 4-Chlor-3-trifluormethyl, steht

Besonders bevorzugt sind substituierte Cyclohexylcarbonsäure-Derivate, worin R³ für Pyridyl, substituiert oder unsubstituiert, oder Phenyl, 2-Fluorphenyl, 3-Fluorphenyl oder 4-Fluorphenyl steht, insbesondere Phenyl.

Bevorzugt sind auch substituierte Cyclohexylcarbonsäure-Derivate, bei denen R⁶ H und R⁷ H oder COOR⁹ bedeutet.

Außerdem bevorzugt sind substituierte Cyclohexylcarbonsäure-Derivate, bei denen R⁵ H bedeutet.

Weiterhin bevorzugt sind substituierte Cyclohexylcarbonsäure-Derivate, bei denen R⁸ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet,
insbesondere

R⁸ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Besonders bevorzugt sind substituierte Cyclohexylcarbonsäure-Derivate, bei denen R⁸ Phenyl oder Indolyl, jeweils einfach oder mehrfach substituiert; bedeutet.

Ganz besonders bevorzugt sind substituierte Cyclohexylcarbonsäure-Derivate aus der Gruppe
2-[(4-Dimethylamino-4-phenyl-cyclohexancarbonyl)-amino]-3-(1H-indol-3-yl)-propansäure-methylester-hydrochlorid; unpolareres Diastereomer
2-[(4-Dimethylamino-4-phenyl-cyclohexancarbonyl)-amino]-3-(1H-indol-3-yl)-propansäure-methylester-hydrochlorid; polareres Diastereomer
4-Dimethylamino-4-phenyl-cyclohexancarbonsäure-(3-phenyl-propyl)-amid-hydrochlorid; unpolareres Diastereomer
4-Dimethylamino-4-phenyl-cyclohexancarbonsäure-(3-phenyl-propyl)-amid-hydrochlorid; polareres Diastereomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten ORL-1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Cyclohexycarbonsäure-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Cyclohexylcarbonsäure-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexylcarbonsäure-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexylcarbonsäure-Derivate verzögert freisetzen. Die erfindungsgemäßen substituierten Cyclohexylcarbonsäure-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Cyclohexylcarbonsäure-Derivats appliziert.

Für alle vorstehenden Formen der erfindungsgemäßen Arzneimittel ist es besonders bevorzugt, wenn das Arzneimittel neben wenigstens einem substituierten Cyclohexylcarbonsäure-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexylcarbonsäure-Derivat als reines Diastereomer und/oder Enantiomer, als Razemat oder als nicht-äquimolare oder äquimolare Mischung der Diastereomere und/oder Enantiomere vor.

Der ORL-1-Rezeptor, aber auch die anderen Opioid-Rezeptoren, wurden insbesondere im Schmerzgeschehen identifiziert. Entsprechend können erfindungsgemäße substituierte Cyclohexylcarbonsäure-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen substituierten Cyclohexylcarbonsäure-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Cyclohexylcarbonsäure-Derivats zur Herstellung eines Arzneimittels zur Behandlung von Angstzuständen, von Stress und mit Stress verbundenen Syndromen, Depressionen, Katalepsie, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Cyclohexylcarbonsäure-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexylcarbonsäure-Derivats, oder eines erfindungsgemäßen Arzneimittels.

Ein weiterer Gegenstand der Erfindung ist ein Verfahren zur Herstellung der erfindungsgemäßen substituierten Cyclohexylcarbonsäure-Derivate wie in der folgenden Beschreibung und Beispielen ausgeführt.

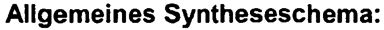

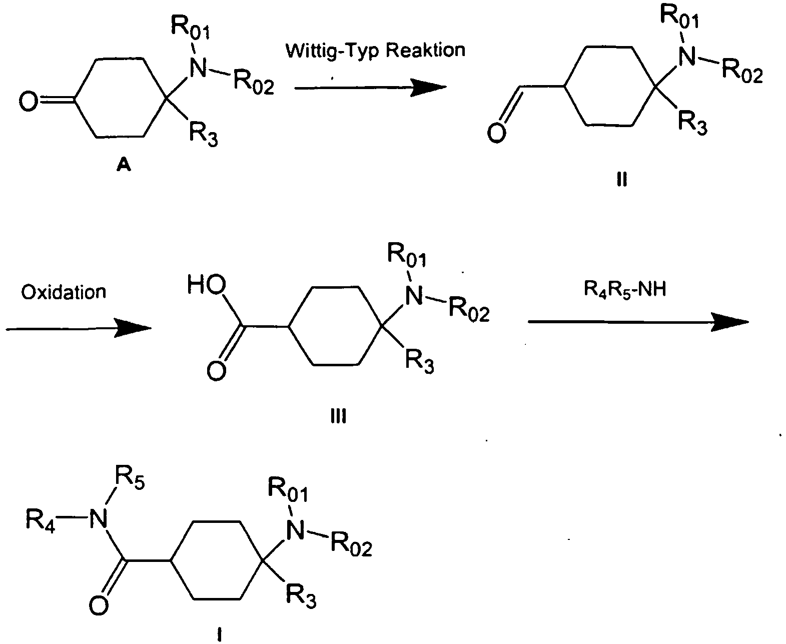

R⁰¹ und R⁰² haben die Bedeutung von R¹ und R² und können zusätzlich die Bedeutung einer Schutzgruppe annehmen.

Die Herstellung geeigneter 4-Aminocyclohexanone gemäß Formel A ist aus der Literatur bekannt (Lednicer et al., J. Med. Chem., 23, 1980, 424-430; WO 0290317).

Ein Alkoxymethyl-phosphoniumsalz, vorzugsweise Methoxymethyl-triphenylphosphonium-chlorid oder Methoxymethyl-triphenylphosphonium-bromid, wird zunächst mit einer starken Base, vorzugsweise Kalium-tert.butylat, Natriumhydrid oder Butyllithium, dann mit einem 4-Aminocyclohexanon gemäß Formel A umgesetzt. Dabei entsteht ein Cyclohexylcarbaldehyd gemäß Formel II.

Der Cyclohexylcarbaldehyd II wird mit einem geeigneten Oxidationsmittel, vorzugsweise mit Kaliumpermanganat, Chrom(VI)oxid oder anderen Chrom(VI)-Salzen, zur korrespondierenden Cyclohexyl-carbonsäure gemäß Formel III oxidiert. Die Carbonsäure gemäß Formel III wird als solche oder als ihr korrespondierendes Hydrochlorid mit einem wasserentziehenden Reagens, bevorzugt mit einem Carbodiimid, besonders bevorzugt mit Dicyclohexyl-carbodiimid, in Gegenwart von einem Aktivierungsreagens, bevorzugt mit 1-Hydroxybenzotriazol, mit einem Amin der Formel R⁴R⁵NH zu dem korrespondierenden Amid gemäß der Formel I umgesetzt.

Gegebenenfalls werden anschließend die Schutzgruppen an R⁰¹ und R⁰² nach dem Fachmann bekannten Methoden abgespalten.

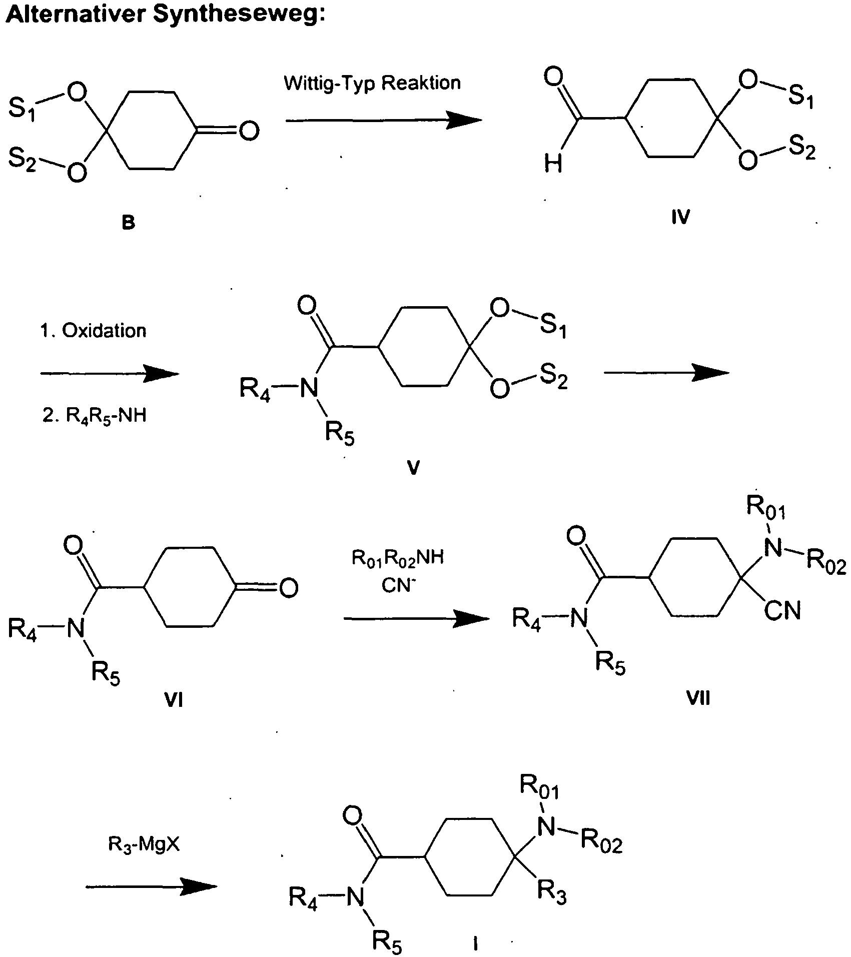

Ein mit den Gruppen S¹ und S², die für Schutzgruppen stehen - beispielsweise substituiertes oder unsubstituiertes Alkyl, insbesondere (CH₂)ₙ mit n = 2-4 - geschütztes Cyclohexan-1,4-dion gemäß Formel B wird in Gegenwart einer starken Base, vorzugsweise Kalium-tert.butylat, Natriumhydrid oder Butyllithium mit einem Alkoxymethyl-phosphoniumsalz, vorzugsweise Methoxymethyl-triphenylphosphonium-chlorid oder Methoxymethyl-triphenylphosphonium-bromid, umgesetzt. Dabei entsteht ein Cyclohexylcarbaldehyd gemäß Formel IV.

Der Cyclohexylcarbaldehyd IV wird mit einem geeigneten Oxidationsmittel, vorzugsweise mit Kaliumpermanganat, Chrom(VI)oxid oder anderen Chrom(VI)-Salzen, zur korrespondierenden Cyclohexyl-Carbonsäure oxidiert. Diese Carbonsäure wird als solche oder als ihr korrespondierendes Hydrochlorid mit einem wasserentziehenden Reagens, bevorzugt mit einem Carbodiimid, besonders bevorzugt mit Dicyclohexyl-carbodiimid, in Gegenwart von einem Aktivierungsreagens, bevorzugt mit 1-Hydroxybenzotriazol, mit einem Amin der Formel R⁴R⁵NH zu dem korrespondierenden Amid gemäß der Formel V umgesetzt.

An der Verbindung gemäß Formel V werden die Schutzgruppen S¹ und S² abgespalten, so dass ein 4-substituiertes Cyclohexanonderivat gemäß Formel VI entsteht.

Die Verbindung gemäß Formel VI wird in Gegenwart einer Verbindung der Formel HNR⁰¹R⁰² mit einem Cyanid, vorzugsweise Kaliumcyanid oder TMSCN, zu einem 4-substituierten 1-Amino-1-cyano-cyclohexanderivat gemäß Formel VII umgesetzt.

Das Aminonitril gemäß Formel VII wird mit metallorganischen Reagenzien, bevorzugt Grignard- oder Organolithiumreagenzien, der Formel Metall-R³ umgesetzt, so dass die erfindungsgemäßen Verbindungen gemäß Formel I entstehen.

Gegebenenfalls werden anschließend die Schutzgruppen an R⁰¹ und R⁰² nach dem Fachmann bekannten Methoden abgespalten.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "THF" Tetrahydrofuran, "DMF" Dimethylformamid, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### 4-Dimethylamino-4-phenyl-cyclohexancarbaldehyd

46 mmol (10,0 g) von 4-Dimethylamino-4-phenylcyclohexanon werden in Toluol gelöst. Diese Lösung wird bei 70 °C zu einer Suspension von 138,1 mmol (47,33 g) Methoxymethyl-triphenylphosphoniumchlorid und 138,1 mmol (25,82 g) Kalium *tert*.-butylat getropft. Man lässt noch einige Stunden bei 70 °C nachrühren, hydrolysiert mit Wasser und extrahiert mit EE. Die vereinigten organischen Phasen werden mit gesättigter NaCl-Lösung gewaschen, über Natriumsulfat getrocknet und eingeengt. Das so erhaltene braune Harz wird an Kieselgel chromatographiert. Man erhält den gewünschten Aldehyd als cis/trans-Gemisch.

### 4-Dimethylamino-4-phenyl-cyclohexancarbonsäure-hydrochlorid

4-Dimethylamino-4-phenyl-cyclohexancarbaldehyd (2,3 g, 10 mmol) wurde in THF (60 ml) gelöst. Kaliumpermanganat (3 g, 19 mmol) wurde bei 50 °C in 50 ml dest. Wasser gelöst, auf RT abgekühlt und zu der Lösung des Aldehyds getropft, so dass die Temperatur unter 40 °C gehalten wurde. Es wurde noch 3 h bei RT gerührt. Ausgeschiedenes Mangandioxid wurde abgesaugt und nacheinander gründlich mit THF und Wasser gewaschen. Die vereinigten Filtrate wurden eingeengt und mit Ether (3 × 15 ml) extrahiert. Danach wurde mit 2M Salzsäure auf pH 1 eingestellt und erneut mit Ether (3 × 20 ml) ausgeschüttelt. Die wässrige Phase wurde zur Trockene eingeengt. Der Rückstand wurde über P₂O₅ getrocknet. Der Feststoff würde in Isopropanol (30 ml) suspendiert, der unlösliche Rückstand abgesaugt, die Mutterlauge eingeengt und bis zum Eintreten einer Trübung mit Ether versetzt. Nach 16-stündigem Kühlen wurde der entstandene Niederschlag abgesaugt, mit Ether gewaschen und getrocknet. Durch fraktionierte Fällung wurden 1,5 g (54 %) von 4-Dimethylamino-4-phenyl-cyclohexancarbonsäure-hydrochlorid mit einem Smp. von 112-130 °C gewonnen.

### 2-[(4-Dimethylamino-4-phenyl-cyclohexancarbonyl)-amino]-3-(1H-indol-3-yl)-propionsäure-methylester-hydrochlorid (Beispiele 1 und 2)

4-Dimethylamino-4-phenyl-cyclohexancarbonsäure-hydrochlorid (568 mg, 2 mmol) und L-Tryptophan-methylester-hydrochlorid (509 mg, 2 mmol) wurden in 5 ml trockenem DMF gelöst und *N*-Methylmorpholin (0,88 ml, 8 mmol) zugesetzt. Nach 10 Minuten wurde 1-Hydroxybenzotriazol (1 g, 8 mmol) zugegeben, auf 0 °C abgekühlt und Dicyclohexylcarbodiimid (1,6 g, 8 mmol) eingetragen. Zur Aufarbeitung wurde der Niederschlag abgesaugt und mit kaltem DMF gewaschen. Das Filtrat wurde mit einer Mischung aus gesättigter NaCl-Lösung (93 ml) und gesättigter Natriumhydrogencarbonatlösung (7 ml) versetzt. Es wurde Ether zugegeben, die Phasen wurden getrennt, die Ether-Phase wurde getrocknet und eingeengt. Aus dem Rückstand (1,3 g) wurde durch Chromatographie ein Teil des unpolareren Diastereoisomers (25 mg, 2,8 %) isoliert. Die wässrige Phase wurde mit EE extrahiert, mit 1 M NaOH auf pH 10 eingestellt und erneut mit EE ausgeschüttelt. Die vereinigten organischen Phasen wurden getrocknet und eingeengt. Aus dem Rückstand (862 mg) wurden durch Chromatographie das unpolarere Diastereoisomer (14 mg, 1,5 %), das polarere Diastereoisomer (62 mg, 7 %) und eine Mischfraktion (75 mg, 8,4 %) isoliert.

Das unpolarere Diastereoisomer (72 mg, 0,16 mmol) wurde in Ethanol (20 ml) gelöst. Bei RT wurde mit 3,3 M ethanolischer HCl (75 µl, 0,24 mmol) versetzt und 2 h gerührt. Das Lösungsmittel wurde abdestilliert und der Rückstand mit Ether verrieben. Der entstandene Feststoff wurde abgesaugt und mit Ether gewaschen. Das unpolarere Diastereoisomer von 2-[(4-Dimethylamino-4-phenyl-cyclohexan-carbonyl)-amino]-3-(1H-indol-3-yl)-propionsäure-methylester-hydrochlorid wurde so als beiger Feststoff (70,2 mg, 91 %) mit einem Smp. von 138-144 °C erhalten (Beispiel 1).

Analog wurde mit dem polareren Diastereoisomer (77 mg, 0,17 mmol) verfahren. Das Hydrochlorid des polareren Diastereoisomers wurde so als beiger Feststoff (83 mg, 99 %) mit einem Smp. von 185-195 °C erhalten (Beispiel 2).

### 4-Dimethylamino-4-phenyl-cyclohexancarbonsäure (3-phenyl-propyl)-amid-hydrochlorid (Beispiele 3 und 4)

4-Dimethylamino-4-phenyl-cyclohexancarbonsäure-hydrochlorid (357 mg, 1,25 mmol) und 3-Phenyl-propylamin-hydrochlorid (170,1 mg, 1,25 mmol) wurden in trockenem DMF (6 ml) gelöst und N-Methylmorpholin (0,28 ml, 2,5 mmol) zugesetzt. Nach 10 Minuten wurde 1-Hydroxybenzotriazol (510 mg, 3,8 mmol) zugegeben, auf 0°C abgekühlt, Dicyclohexylcarbodiimid (778 mg, 3,8 mmol) eingetragen und 6 Tage bei RT gerührt. Zur Aufarbeitung wurde das Reaktionsgemisch 2h gekühlt, der Feststoff abgesaugt und mit kaltem DMF gewaschen. Das Filtrat wurde mit einer gekühlten Mischung aus gesättigter NaCl-Lösung (46 ml) und gesättigter Natriumhydrogencarbonat-Lösung (4 ml) versetzt. Es wurde Ether zugegeben und die Phasen getrennt. Die Ether-Phase wurde getrocknet und eingeengt. Die wässrige Phase wurde mit Dichlormethan (4 × 10 ml) extrahiert. Die Dichlormethan-Extrakte wurden getrocknet und eingeengt. Die Extraktionsrückstände wurden vereinigt und durch Chromatographie gereinigt. So wurden das unpolarere Diastereoisomer (21 mg, 4 %), ein Gemisch (30 mg, 7 %) und das polarere Diastereoisomer (109 mg, 24 %) erhalten.

Das unpolarere Diastereoisomer (20 mg, 0,05 mmol) wurde in Ethanol (2 ml) und Methylethylketon (2 ml) gelöst. Bei RT wurde mit 3,3 M ethanolischer HCl (25 µl, 0,08 mmol) versetzt und 2 h gerührt. Die Lösungsmittel wurden abdestilliert und der Rückstand mit Ether verrieben. Der entstandene Feststoff wurde abgesaugt und mit Ether (2 × 1 ml) gewaschen. Das unpolarere Diastereoisomer von 4-Dimethylamino-4-phenyl-cyclohexancarbonsäure (3-phenyl-propyl)-amid-hydrochlorid wurde so als hellgelber Feststoff (19 mg, 88 %) mit einem Smp. von 100-105 °C erhalten (Beispiel 3).

Mit dem polaren Diastereomer (105 mg, 0,29 mmol) wurde analog verfahren. Das Hydrochlorid des polaren Diastereomers wurde so als farbloser Feststoff (115 mg, 96 %) mit einem Smp. von 112-115 °C erhalten (Beispiel 4).

### Untersuchungen zur Wirksamkeit der erfindungsgemäßen Verbindungen:

### Messung der ORL1-Bindung

Die Cyclohexan-Derivate der allgemeinen Formel I wurden in einem Rezeptor-bindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten Cyclohexylcarbonsäure-Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglütinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

### Messung der Serotonin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### Messung der Noradrenalin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert:

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Beispielhaft wurden die folgenden Bindungsdaten bestimmt:

| Beispiel-Nummer | ORL1 % [1µM] |
|---|---|
| **1** | 34.00 |
| **3** | 30.00 |

| Beispiel-Nummer | ORµNal % [1µM] |
|---|---|
| **1** | 55 |
| **2** | 34 |
| **3** | 70.5 |
| **4** | 59 |

| Beispiel-Nummer | 5HT-Uptake % Hemmung [10µM] |
|---|---|
| **1** | 74 |
| **2** | 56 |
| **3** | 88 |
| **4** | 85 |

| Beispiel-Nummer | NA-Uptake % Hemmung [10µM] |
|---|---|
| **1** | 30 |
| **3** | 65 |
| **4** | 71 |

### Parenterale Lösung eines erfindungsgemäßen substituierten Cyclohexylcarbonsäure-Derivats

38 g eines der erfindungsgemäßen substituierten Cyclohexylcarbonsäure-Derivate, hier Beispiel 1, wird in 1 I Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Cyclohexylcarbonsäure-Derivate-Derivate der allgemeinen Formel I, 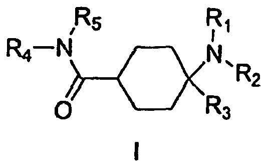 , worin
R¹ und R², unabhängig voneinander für H; CHO; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R³ für Cyclopentyl, Cyclohexyl, Naphthyl, Thiophenyl, Benzothiophenyl, Furyl, Benzofuranyl, Benzodioxolanyl, Indolyl, Indanyl, Benzodioxanyl oder Pyridyl, jeweils unsubstituiert oder einfach oder mehrfach substituiert; über eine gesättigte, unverzweigte C₁₋₂-Alkyl-Gruppe gebundenen C₅₋₆-Cycloalkyl,
Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-ethylphenyl, 4-Methylphenyl, 2-Methoxyphenyl, 3-Methoxyphenyl, 4-Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthlophenyl, 5-Fluor-2-methoxyphenyl, 4-Chlor-3-trifluormethyl oder 4-Brom-2-methylphenyl steht;
R⁴ für für-(CR⁶R⁷)ₙR⁸ steht,
wobei n 0, 1, 2, 3, 4, 5 oder 6 bedeutet
R⁶ H oder C₁₋₅-Alkyl, gesättigt oder ungesättigt, verzweigt oder unverzweigt, unsubstituiert oder einfach oder mehrfach substituiert, bedeutet
R⁷ H, C₁₋₅-Alkyl, gesättigt oder ungesättigt, verzweigt oder unverzweigt, unsubstituiert oder einfach oder mehrfach substituiert, oder COOR⁹ bedeutet,
oder R⁶ und R⁷ einen Ring (CH₂)ₖCHR⁸(CH₂)ₘ bilden, mit k = 1, 2, 3 und m=1,2
R⁸ C₃₋₈-Cycloalkyl, Aryl oder Heteroaryl, jeweils unsubstituiert oder einfach oder mehfach substituiert, bedeutet,
R⁹ H oder C₁₋₅-Alkyl, gesättigt oder ungesättigt, verzweigt oder unverzweigt, unsubstituiert oder einfach oder mehrfach substituiert, bedeutet
R⁵ für H oder für-(CH₂)ₗR⁸ steht,
wobei I für 1, 2 oder 3 steht,
oder zusammen mit R⁴ für CH₂CH₂OCH₂CH₂ oder CH₂CH₂NR¹¹CH₂CH₂ stehen,
wobei R¹¹H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte Cyclohexylcarbonsäure-Derivate gemäß Anspruch 1, **dadurch gekennzeichnet, dass** R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₅-Alkyl, gesättigt oder ungesättigt, verzweigt oder unverzweigt, einfach oder mehrfach substituiert oder unsubstituiert, bedeutet.

3. Substituierte Cyclohexylcarbonsäure-Derivate gemäß Anspruch 1, **dadurch gekennzeichnet, dass** dass R¹ und R² unabhängig voneinander für CH₃ oder H stehen, wobei R¹ und R² nicht gleichzeitig H bedeuten.

4. Substituierte Cyclohexylcarbonsäure -Derivate gemäß einem der Ansprüche 1 bis 3, **dadurch gekennzeichnet, dass** R³ für Naphthyl, Thiophenyl oder Pyridyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Thiophenyl, Pyridyl, jeweils unsubstituiert oder einfach oder mehrfach substituiert; Phenyl, 2-Fluorphenyl, 3-Fluorphenyl, 4-Fluorphenyl, 2-Chlorphenyl, 4-Chlorphenyl, 3-Chlorphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 4-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl oder 4-Chlor-3-trifluormethyl, steht.

5. Substituierte Cyclohexylcarbonsäure-Derivate gemäß einem der Ansprüche 1-3, **dadurch gekennzeichnet, dass** R³ für Pyridyl, Phenyl, 3-Fluorphenyl oder 4-Fluorphenyl steht.

6. Substituierte Cyclohexylcarbonsäure-Derivate gemäß einem der Ansprüche 1-3, **dadurch gekennzeichnet, dass** R³ Phenyl bedeutet.

7. Substituierte Cyclohexylcarbonsäure-Derivate gemäß einem der Ansprüche 1-6, **dadurch gekennzeichnet, dass** R⁶ H und R⁷ H oder COOR⁹ bedeutet.

8. Substituierte Cyclohexylcarbonsäure-Derivate gemäß einem der Ansprüche 1-7. **dadurch gekennzeichnet, dass** R⁵ H bedeutet.

9. Substituierte Cyclohexylcarbonsäure-Derivate gemäß einem der Ansprüche 1-8, **dadurch gekennzeichnet, dass** R⁸ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet,
insbesondere
R⁸ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

10. Substituierte Cyclohexylcarbonsäure-Derivate gemäß einem der Ansprüche 1-8, **dadurch gekennzeichnet, dass** R⁸ Phenyl oder Indolyl, jeweils einfach oder mehrfach substituiert, bedeutet.

11. Substituierte Cyclohexylcarbonsäure-Derivate gemäß einem der Ansprüche 1-10 aus der Gruppe
2-[(4-Dimethylamino-4-phenyl-cyclohexancarbonyl)-amino]-3-(1H-indol-3-yl)-propansäure-methylester-hydrochlorid; unpolareres Diastereomer
2-[(4-Dimethylamino-4-phenyl-cyclohexancarbonyl)-amino]-3-(1H-indol-3-yl)-propansäure-methylester-hydrochlorid; polareres Diastereomer
4-Dimethylamino-4-phenyl-cyclohexancarbonsäure-(3-phenyl-propyl)-amid-hydrochlorid; unpolareres Diastereomer
4-Dimethylamino-4-phenyl-cyclohexancarbonsäure-(3-phenyl-propyl)-amid-hydrochlorid; polareres Diastereomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

12. Verfahren zur Herstellung von substituierten Cyclohexylcarbonsäure-Derivate gemäß einem der Ansprüche 1-11, **dadurch gekennzeichnet, dass** Alkoxymethyl-phosphoniumsalze in Gegenwart einer starken Base mit einem 4-Aminocyclohexanon umgesetzt, anschließend mit einem geeigneten Oxidationsmittel, vorzugsweise mit Kaliumpermanganat, Chrom(VI)oxid oder anderen Chrom(VI)-Salzen, oxidiert und in Gegenwart eines wasserentziehenden Reagenzes, vorzugsweise eines Carbodiimids, und eines Aktivierungsreagenzes, vorzugsweise 1-Hydroxybenzotriazol, mit einem Amin der Formel R⁴R⁵NH umgesetzt werden.

13. Verfahren zur Herstellung von substituierten Cyclohexylcarbonsäure-Derivate gemäß einem der Ansprüche 1-11, **dadurch gekennzeichnet, dass** geschützte Cyclohexan-1,4-dione in Gegenwart einer starken Base mit einem Alkoxymethyl-phosphoniumsalz umgesetzt, mit einem geeigneten Oxidationsmittel, vorzugsweise Kaliumpermanganat, Chrom(VI)oxid oder andere Chrom(VI)-Salzen, oxidiert, in Gegenwart eines wasserentziehenden Reagenzes, vorzugsweise eines Carbodiimids, und eines Aktivierungsreagenzes, vorzugsweise 1-Hydroxybenzotriazol, mit einem Amin der Formel R⁴R⁵NH umgesetzt, die Schutzgruppen abgespalten, in Gegenwart einer Verbindung der Formel HNR⁰¹R⁰² mit einem Cyanid umgesetzt, und mit metallorganischen Reagenzien, bevorzugt Grignard- oder Organolithiumreagenzien, der Formel Metall-R³, umgesetzt werden.

14. Arzneimittel enthaltend mindestens ein substituiertes Cyclohexylcarbonsäure - Derivat gemäß einem der Ansprüche 1 bis 11 sowie gegebenenfalls geeignete Zusatz- und/oder Hilfsstoffe und/oder gegebenenfalls weitere Wirkstoffe.

15. Verwendung eines substituierten Cyclohexylcarbonsäure-Derivats gemäß einem der Ansprüche 1 bis 11 zur Herstellung eines Arzneimittels zur Behandlung von Schmerz, insbesondere von akutem, viszeralem, neuropathischem oder chronischem Schmerz.

16. Verwendung eines substituierten Cyclohexylcarbonsäure-Derivats gemäß einem der Ansprüche 1 bis 11 zur Herstellung eines Arzneimittels zur Behandlung von Angstzuständen, von Stress und mit Stress verbundenen Syndromen, Depressionen, Katalepsie, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Substituted cyclohexylcarboxylic acid derivatives of general formula I, 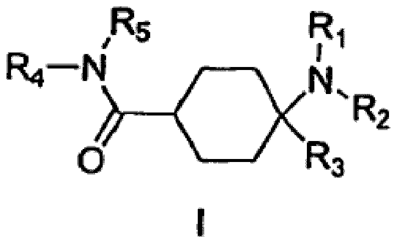 wherein
R¹ and R² independently of one another represent H; CHO; respectively saturated or unsaturated, branched or unbranched, singly or multiply substituted or unsubstituted C₁₋₅ alkyl; respectively saturated or unsaturated, singly or multiply substituted or unsubstituted C₃₋₈ cycloalkyl; or respectively singly or multiply substituted or unsubstituted aryl, C₃₋₈ cycloalkyl or heteroaryl bound by C₁₋₃ alkyl;
or the radicals R¹ and R² together represent CH₂CH₂OCH₂CH₂,
CH₂CH₂NR¹⁰CH₂CH₂ or (CH₂)₃₋₆
wherein R¹⁰ represents H; respectively saturated or unsaturated, branched or unbranched, singly or multiply substituted or unsubstituted C₁₋₅ alkyl; respectively saturated or unsaturated, singly or multiply substituted or unsubstituted C₃₋₈ cycloalkyl; respectively singly or multiply substituted or unsubstituted aryl or heteroaryl; or respectively singly or multiply substituted or unsubstituted aryl, C₃₋₈ cycloalkyl or heteroaryl bound by C₁₋₃ alkyl;
R³ represents respectively unsubstituted or singly or multiply substituted cyclopentyl, cyclohexyl, naphthyl, thiophenyl, benzothiophenyl, furyl, benzofuranyl, benzodioxolanyl, indolyl, indanyl, benzodioxanyl or pyridyl; respectively unsubstituted or singly or multiply substituted C₅₋₆ cycloalkyl, phenyl, napthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl bound by a saturated, unbranched C₁₋₂ alkyl group; phenyl, 2-fluorophenyl, 3-fluorophenyl, 4-fluorophenyl, 2-chlorophenyl, 4-chlorophenyl, 3-chlorophenyl, 2-bromophenyl, 3-bromophenyl, 4-bromophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-ethylphenyl, 3-ethylphenyl, 4-ethylphenyl, 2-ethoxyphenyl, 3-ethoxyphenyl, 4-ethoxyphenyl, 2-hydroxyphenyl, 3-hydroxyphenyl, 4-hydroxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert.-butylphenyl, 4-fluoro-3-chlorophenyl, 4-bromo-3-fluorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl, 5-fluoro-2-methoxyphenyl, 4-chloro-3-trifluoromethyl or 4-bromo-2-methylphenyl;
R⁴ represents -(CR⁶R⁷)ₙR⁸,
wherein n represents 0, 1, 2, 3, 4, 5 or 6
R⁶ represents H or C₁₋₅ alkyl, saturated or unsaturated, branched or unbranched, unsubstituted or singly or multiply substituted
R⁷ represents H, saturated or unsaturated, branched or unbranched, unsubstituted or singly or multiply substituted C₁₋₅ alkyl, or COOR⁹,
or R⁶ and R⁷ form a ring (CH₂)ₖCHR⁸(CH₂)ₘ, where k = 1, 2, 3 and m = 1, 2
R⁸ represents respectively unsubstituted or singly or multiply substituted C₃₋₈ cycloalkyl, aryl or heteroaryl,
R⁹ represents H or saturated or unsaturated, branched or unbranched, unsubstituted or singly or multiply substituted C₁₋₅ alkyl
R⁵ represents H or -(CH₂)ₗR⁸,
wherein I represents 1, 2 or 3,
or together with R⁴ represents CH₂CH₂OCH₂CH₂ or CH₂CH₂NR¹¹CH₂CH₂,
wherein R¹¹ represents H; respectively saturated or unsaturated, branched or unbranched, singly or multiply substituted or unsubstituted C₁₋₅ alkyl; respectively saturated or
unsaturated, singly or multiply substituted or unsubstituted C₃₋₈ cycloalkyl; respectively singly or multiply substituted or unsubstituted aryl or heteroaryl; or respectively singly or multiply substituted or unsubstituted aryl, C₃₋₈ cycloalkyl or heteroaryl bound by C₁₋₃ alkyl;
in the form of the racemate; the enantiomers, diastereomers, mixtures of the enantiomers or diastereomers or an individual enantiomer or diastereomer; the bases and/or salts of physiologically acceptable acids or cations.

2. Substituted cyclohexylcarboxylic acid derivatives according to claim 1, **characterised in that** R¹ and R² independently of one another represent H; saturated or unsaturated, branched or unbranched, singly or multiply substituted or unsubstituted C₁₋₅ alkyl.
or the radicals R¹ and R² together form a ring and represent CH₂CH₂OCH₂CH₂, CH₂CH₂NR¹⁰CH₂CH₂ or (-CH₂)₃₋₆,
wherein R¹⁰ represents H; saturated or unsaturated, branched or unbranched, singly or multiply substituted or unsubstituted C₁₋₅ alkyl.

3. Substituted cyclohexylcarboxylic acid derivatives according to claim 1, **characterised in that** R¹ and R² independently of one another represent CH₃ or H, wherein R¹ and R² do not simultaneously represent H.

4. Substituted cyclohexylcarboxylic acid derivatives according to any one of claims 1 to 3, **characterised in that** R³ represents respectively unsubstituted or singly or multiply substituted naphthyl, thiophenyl, or pyridyl; respectively unsubstituted or singly or multiply substituted C₅₋₆cycloalkyl, phenyl, naphthyl, thiophenyl, pyridyl bound by a saturated, unbranched C₁₋₂ alkyl group; phenyl, 2-fluorophenyl, 3-fluorophenyl, 4-fluorophenyl, 2-chlorophenyl, 4-chlorophenyl, 3-chlorophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 4-trifluoromethylphenyl, 2-ethylphenyl, 3-ethylphenyl, 4-ethylphenyl, 2-ethoxyphenyl, 3-ethoxyphenyl, 4-ethoxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert.-butylphenyl, 4-fluoro-3-chlorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl, or 4-chloro-3-trifluoromethyl.

5. Substituted cyclohexylcarboxylic acid derivatives according to any one of claims 1 to 3, **characterised in that** R³ represents pyridyl, phenyl, 3-fluorophenyl or 4-fluorophenyl.

6. Substituted cyclohexylcarboxylic acid derivatives according to any one of claims 1 to 3, **characterised in that** R³ represents phenyl.

7. Substituted cyclohexylcarboxylic acid derivatives according to any one of claims 1 to 6, **characterised in that** R⁶ represents H and R⁷ represents H or COOR⁹.

8. Substituted cyclohexylcarboxylic acid derivatives according to any one of claims 1 to 7, **characterised in that** R⁵ represents H.

9. Substituted cyclohexylcarboxylic acid derivatives according to any one of claims 1 to 8, **characterised in that** R⁸ represents respectively unsubstituted or singly or multiply substituted cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzyl[1,2,5]thiazolyl or 1.2-dihydroacenaphtenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl,
in particular
R⁸ represents respectively unsubstituted or singly or multiply substituted cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl.

10. Substituted cyclohexylcarboxylic acid derivatives according to any one of claims 1 to 8, **characterised in that** R⁸ represents respectively singly or multiply substituted phenyl or indolyl.

11. Substituted cyclohexylcarboxylic acid derivatives according to any one of claims 1 to 10 from the group comprising
2-[(4-dimethylamino-4-phenyl-cyclohexanecarbonyl)-amino]-3-(1H-indol-3-yl)-propionic acid-methylester-hydrochloride; non-polar diastereomer
2-[(4-dimethylamino-4-phenyl-cyclohexanecarbonyl)-amino]-3-(1 H-indol-3-yl)-propionic acid-methylester-hydrochloride; polar diastereomer
4-dimethylamino-4-phenyl-cyclohexanecarboxylic acid (3-phenyl-propyl)amide hydrochloride; non-polar diastereomer
4-dimethylamino-4-phenyl-cyclohexanecarboxylic acid (3-phenyl-propyl)-amide-hydrochloride; polar diastereomer
in the form of the racemate; the enantiomers, diastereomers, mixtures of the enantiomers or diastereomers or an individual enantiomer or diastereomer; the bases and/or salts of physiologically acceptable acids or cations.

12. Process for producing substituted cyclohexylcarboxylic acid derivatives according to any one of claims 1 to 11, **characterised in that** alkoxymethyl-phosphonium salts are reacted in the presence of a strong base with a 4-aminocyclohexanone, subsequently oxidised with a suitable oxidising agent, preferably with potassium permanganate, chromium(VI)oxide or other chromium(VI) salts, and reacted in the presence of a dehydrating reagent, preferably a carbodiimide, and an activation reagent, preferably 1-hydroxybenzotriazole, with an amine of formula R⁴R⁵NH.

13. Process for producing substituted cyclohexylcarboxylic acid derivatives according to any one of claims 1 to 11, **characterised in that** protected cyclohexane-1,4-dione is reacted in the presence of a strong base with an alkoxymethyl phosphonium salt, oxidised with a suitable oxidising agent, preferably potassium permanganate, chromium(VI)oxide or other chromium(VI) salts, reacted in the presence of a dehydrating reagent, preferably a carbodiimide, and an activation reagent, preferably 1-hydroxybenzotriazole, with an amine of formula R⁴R⁵NH, the protecting group is cleaved, reacted with a cyanide in the presence of a compound of formula HNR⁰¹R⁰² , and reacted with organometallic reagents, preferably Grignard or organolithium reagents, of the formula metal-R³.

14. Pharmaceutical composition containing at least one substituted cyclohexylcarboxylic acid derivative according to any one of claims 1 to 11 and optionally suitable additives and/or auxiliaries and/or optionally further active ingredients.

15. Use of a substituted cyclohexylcarboxylic acid derivative according to any one of claims 1 to 11 for producing a pharmaceutical composition for the treatment of pain, in particular acute, visceral, neuropathic or chronic pain.

16. Use of a substituted cyclohexylcarboxylic acid derivative according to any one of claims 1 to 11 for producing a pharmaceutical composition for the treatment of anxiety, stress and stress-related syndromes, depression, catalepsy, epilepsy, Alzheimer's disease, senile dementia, general cognitive dysfunction, learning and memory difficulties (as a nootropic), withdrawal symptoms, alcohol- and/or drug-and/or medicines abuse and/or dependency, sexual dysfunction, cardiovascular diseases, hypotension, hypertension, tinnitus, pruritus, migraine, hearing difficulties, deficient intestinal motility, impaired nutrient absorption, anorexia, obesity, locomotive disorders, diarrhoea, cachexia, urinary incontinence or as a muscle relaxant, anti-convulsive or anaesthetic or for co-administration in treatment with an opioid analgesic or anaesthetic, for diuresis or anti-natriuresis, anxiolysis, for modulation of motor activity, for modulation of neurotransmitter release and treatment of neurodegenerative diseases associated therewith, for the treatment of withdrawal symptoms and/or for reducing the potential for addiction to opioids.

## Revendications

1. Dérivés substitués de l'acide cyclohexylcarboxylique de formule générale I 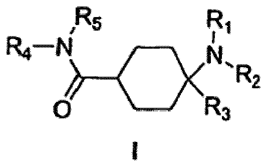 dans laquelle
R¹ et R² représentent indépendamment l'un de l'autre H ; CHO ; alkyle en C₁₋₅ à chaque fois saturé ou insaturé, ramifié ou non ramifié, substitué une ou plusieurs fois ou non substitué ; cycloalkyle en C₃₋₈, à chaque fois saturé ou insaturé, substitué ou une plusieurs fois ou non substitué ; ou aryle, cycloalkyle en C₃₋₈ ou hétéroaryle, à chaque fois substitué une ou plusieurs fois ou non substitué, relié par alkyle en C₁₋₃ ;
ou les radicaux R¹ et R² représentent ensemble CH₂CH₂OCH₂CH₂, CH₂CH₂NR¹⁰CH₂CH₂ ou (CH₂) ₃₋₆,
R¹⁰ signifiant H ; alkyle en C₁₋₅, à chaque fois saturé ou insaturé, ramifié ou non ramifié, substitué une ou plusieurs fois ou non substitué ; cycloalkyle en C₃₋₈, à chaque fois saturé ou insaturé, substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle, à chaque fois substitué une ou plusieurs fois ou non substitué, relié par alkyle en C₁₋₃ ;
R³ représente cyclopentyle, cyclohexyle, naphtyle, thiophényle, benzothiophényle, furyle, benzofuranyle, benzodioxolanyle, indolyle, indanyle, benzodioxanyle ou pyridyle, à chaque fois non substitué ou substitué une ou plusieurs fois ; cycloalkyle en C₅₋₆, phényle, naphtyle, anthracényle, thiophényle, benzothiophényle, pyridyle, furyle, benzofuranyle, benzodioxolanyle, indolyle, indanyle, benzodioxanyle, pyrrolyle, pyrimidyle ou pyrazinyle, à chaque fois non substitué ou substitué une ou plusieurs fois, relié par un groupe alkyle en C₁₋₂ saturé, non ramifié ; phényle, 2-fluorophényle, 3-fluorophényle, 4-fluorophényle, 2-chlorophényle, 4-chlorophényle, 3-chlorophényle, 2-bromophényle, 3-bromophényle, 4-bromophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 4-trifluorométhylphényle, 2-éthylphényle, 3-éthylphényle, 4-éthylphényle, 2-éthoxyphényle, 3-éthoxyphényle, 4-éthoxyphényle, 2-hydroxyphényle, 3-hydroxyphényle, 4-hydroxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert.-butylphényle, 4-fluoro-3-chlorophényle, 4-bromo-3-fluorophényle, 3,5-bis(trifluorométhyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle, 5-fluoro-2-méthoxyphényle, 4-chloro-3-trifluorophényle ou 4-bromo-2-méthylphényle ;
R⁴ représente-(CR⁶R⁷)ₙR⁸,
n signifiant 0, 1, 2, 3, 4, 5 ou 6,
R⁶ signifiant H ou alkyle en C₁₋₅, saturé ou insaturé, ramifié ou non ramifié, non substitué ou substitué une ou plusieurs fois,
R⁷ signifiant H, alkyle en C₁₋₅, saturé ou insaturé, ramifié ou non ramifié, non substitué ou substitué une ou plusieurs fois, ou COOR⁹,
ou R⁶ et R⁷ formant un cycle (CH₂)ₖCHR⁸(CH₂)ₘ, avec k = 1, 2, 3 et m = 1, 2,
R⁸ signifiant cycloalkyle en C₃₋₈, aryle ou hétéroaryle, à chaque fois non substitué ou substitué une ou plusieurs fois,
R⁹ signifiant H ou alkyle en C₁₋₅, saturé ou insaturé, ramifié ou non ramifié, non substitué ou substitué une ou plusieurs fois,
R⁵ représente H ou -(CH₂)ₗR⁸,
l représentant 1, 2 ou 3,
ou représente conjointement avec R⁴ CH₂CH₂OCH₂CH₂ ou CH₂CH₂NR¹¹CH₂CH₂,
R¹¹ signifiant H ; alkyle en C₁₋₅, à chaque fois saturé ou insaturé, ramifié ou non ramifié, substitué une ou plusieurs fois ou non substitué ; cycloalkyle en C₃₋₈, à chaque fois saturé ou insaturé, substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle, à chaque fois substitué une ou plusieurs fois ou non substitué, relié par alkyle en C₁₋₃ ;
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement compatibles.

2. Dérivés substitués de l'acide cyclohexylcarboxylique selon la revendication 1, **caractérisés en ce que** R¹ et R² représentent indépendamment l'un de l'autre H ; alkyle en C₁₋₅, saturé ou insaturé, ramifié ou non ramifié, substitué une ou plusieurs fois ou non substitué ;
ou les radicaux R¹ et R² forment ensemble un cycle et signifient CH₂CH₂OCH₂CH₂, CH₂CH₂NR¹⁰CH₂CH₂ ou (CH₂)₃₋₆,
R¹⁰ signifiant H ; alkyle en C₁₋₅, saturé ou insaturé, ramifié ou non ramifié, substitué une ou plusieurs fois ou non substitué.

3. Dérivés substitués de l'acide cyclohexylcarboxylique selon la revendication 1, **caractérisés en ce que** R¹ et R² représentent indépendamment l'un de l'autre CH₃ ou H, R¹ et R² ne signifiant pas simultanément H.

4. Dérivés substitués de l'acide cyclohexylcarboxylique selon l'une quelconque des revendications 1 à 3, **caractérisés en ce que** R³ représente naphtyle, thiophényle ou pyridyle, à chaque fois non substitué ou substitué une ou plusieurs fois, cycloalkyle en C₅₋₆, phényle, naphtyle, thiophényle, pyridyle, à chaque fois non substitué ou substitué une ou plusieurs fois, relié par un groupe alkyle en C₁₋₂ saturé, non ramifié ; phényle, 2-fluorophényle, 3-fluorophényle, 4-fluorophényle, 2-chlorophényle, 4-chlorophényle, 3-chlorophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 4-trifluorométhylphényle, 2-éthylphényle, 3-éthylphényle, 4-éthylphényle, 2-éthoxyphényle, 3-éthoxyphényle, 4-éthoxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert.-butylphényle, 4-fluoro-3-chlorophényle, 3,5-bis(trifluorométhyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle ou 4-chloro-3-trifluorométhyle.

5. Dérivés substitués de l'acide cyclohexylcarboxylique selon l'une quelconque des revendications 1 à 3, **caractérisés en ce que** R³ représente pyridyle, phényle, 3-fluorophényle ou 4-fluorophényle.

6. Dérivés substitués de l'acide cyclohexylcarboxylique selon l'une quelconque des revendications 1 à 3, **caractérisés en ce que** R³ signifie phényle.

7. Dérivés substitués de l'acide cyclohexylcarboxylique selon l'une quelconque des revendications 1 à 6, **caractérisés en ce que** R⁶ signifie H et R⁷ signifie H ou COOR⁹.

8. Dérivés substitués de l'acide cyclohexylcarboxylique selon l'une quelconque des revendications 1 à 7, **caractérisés en ce que** R⁵ signifie H.

9. Dérivés substitués de l'acide cyclohexylcarboxylique selon l'une quelconque des revendications 1 à 8, **caractérisés en ce que** R⁸ signifie cyclobutyle, cyclopropyle, cyclopentyle, cyclohexyle, cycloheptyle, cyclooctyle, anthracényle, indolyle, naphtyle, benzofuranyle, benzothiophényle, indanyle, benzodioxanyle, benzodioxolanyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furanyle, benzofuranyle, pyrazolinonyle, oxopyrazolinonyle, dioxolanyle, adamantyle, pyrimidinyle, quinolinyle, isoquinolinyle, phtalazinyle ou quinazolinyle, à chaque fois non substitué ou substitué une ou plusieurs fois,
notamment
R⁸ signifie cyclopentyle, cyclohexyle, cycloheptyle, cyclooctyle, anthracényle, indolyle, naphtyle, benzofuranyle, benzothiophényle, indanyle, benzodioxanyle, benzodioxolanyle, acénaphtyle, carbazolyle, phényle, thiophényle, furyle, pyridyle, pyrrolyle, pyrazinyle ou pyrimidyle, à chaque fois non substitué ou substitué une ou plusieurs fois.

10. Dérivés substitués de l'acide cyclohexylcarboxylique selon l'une quelconque des revendications 1 à 8, **caractérisés en ce que** R⁸ signifie phényle ou indolyle, à chaque fois substitué une ou plusieurs fois.

11. Dérivés substitués de l'acide cyclohexylcarboxylique selon l'une quelconque des revendications 1 à 10, du groupe constitué par :
le chlorhydrate de l'ester méthylique de l'acide 2-[(4-diméthylamino-4-phényl-cyclohexanecarbonyl)-amino]-3-(1H-indol-3-yl)-propanoïque ; diastéréomère apolaire le chlorhydrate de l'ester méthylique de l'acide 2-[(4-4diméthylamino-4-phényl-cyclohexanecarbonyl)-amino]-3-(1H-indol-3-yl)-propanoïque ; diastéréomère polaire le chlorhydrate du (3-phényl-propyl)-amide de l'acide 4-diméthylamino-4-phényl-cyclohexanecarboxylique ;
diastéréomère apolaire le chlorhydrate du (3-phényl-propyl)-amide de l'acide 4-diméthylamino-4-phényl-cyclohexanecarboxylique ;
diastéréomère polaire sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement compatibles.

12. Procédé de fabrication de dérivés substitués de l'acide cyclohexylcarboxylique selon l'une quelconque des revendications 1 à 11, **caractérisé en ce que** des sels d'alcoxyméthyl-phosphonium sont mis en réaction en présence d'une base forte avec une 4-aminocyclohexanone, puis oxydés avec un oxydant approprié, de préférence avec le permanganate de potassium, l'oxyde de chrome (VI) ou d'autres sels de chrome (VI), et mis en réaction en présence d'un réactif déshydratant, de préférence d'un carbodiimide, et d'un réactif activateur, de préférence le 1-hydroxybenzotriazole, avec une amine de formule R⁴R⁵NH.

13. Procédé de fabrication de dérivés substitués de l'acide cyclohexylcarboxylique selon l'une quelconque des revendications 1 à 11, **caractérisé en ce que** des cyclohexane-1,4-diones protégées sont mises en réaction en présence d'une base forte avec un sel d'alcoxyméthyl-phosphonium, oxydées avec un oxydant approprié, de préférence le permanganate de potassium, l'oxyde de chrome (VI) ou d'autres sels de chrome (VI) , mises en réaction en présence d'un réactif déshydratant, de préférence d'un carbodiimide, et d'un réactif activateur, de préférence le 1-hdyroxybenzotriazole, avec une amine de formule R⁴R⁵NH, débarrassées des groupes protecteurs par clivage, mises en réaction en présence d'un composé de formule HNR⁰¹R⁰² avec un cyanure, et mises en réaction avec des réactifs métallo-organiques, de préférence des réactifs de Grignard ou d'organolithium, de formule métal-R³.

14. Médicament contenant au moins un dérivé substitué de l'acide cyclohexylcarboxylique selon l'une quelconque des revendications 1 à 11, ainsi qu'éventuellement des additifs et/ou adjuvants appropriés et/ou éventuellement des agents actifs supplémentaires.

15. Utilisation d'un dérivé substitué de l'acide cyclohexylcarboxylique selon l'une quelconque des revendications 1 à 11 pour la fabrication d'un médicament pour le traitement de la douleur, notamment de la douleur aigue, viscérale, neuropathique ou chronique.

16. Utilisation d'un dérivé substitué de l'acide cyclohexylcarboxylique selon l'une quelconque des revendications 1 à 11 pour la fabrication d'un médicament pour le traitement des états d'anxiété, du stress et des syndromes associés avec le stress, des dépressions, de la catalepsie, de l'épilepsie, de la maladie d'Alzheimer, de la démence sénile, des dysfonctionnements cognitifs généraux, des troubles de l'apprentissage et de la mémoire (en tant que nootropique), des phénomènes de sevrage, de l'abus et/ou de la dépendance à l'alcool et/ou à des drogues et/ou à des médicaments, des dysfonctionnements sexuels, des maladies cardiovasculaires, de l'hypotension, de l'hypertension, des acouphènes, du prurit, de la migraine, des déficiences auditives, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthétique ou pour la co-administration lors d'un traitement avec un analgésique opioïde ou avec un anesthétique, pour la diurèse ou l'antinatriurèse, l'anxiolyse, pour la modulation de l'activité motrice, pour la modulation de la distribution des neurotransmetteurs et le traitement de maladies neurodégénératives associées, pour le traitement de phénomènes de sevrage et/ou pour la réduction de l'effet de dépendance des opioïdes.
